# EUROPÄISCHE PATENTSCHRIFT

(11) **EP 1 246 566 B1**
(45) Veröffentlichungstag und Bekanntmachung des Hinweises auf die Patenterteilung: **15.12.2004**
(21) Anmeldenummer: 00900201.5
(22) Anmeldetag: 14.01.2000
(51) Int. Cl.: A61B 6/00

(54) **VORRICHTUNG ZUR KONTROLLIERTEN BEWEGUNG EINES MEDIZINISCHEN GERÄTS**
DEVICE FOR MOVING A MEDICAL APPARATUS IN A CONTROLLED MANNER
DISPOSITIF POUR DEPLACER DE FA ON MAITRISEE UN APPAREIL MEDICAL

(43) Veröffentlichungstag der Anmeldung: 09.10.2002
(73) Patentinhaber: AO-Entwicklungsinstitut Davos, 7270 Davos (CH); Suhm, Norbert, 79576 Wil-Haltingen (DE); Messmer, Peter, 4104 Oberwil (CH); Regazzoni, Pietro, 4054 Basel (CH); Müller, Paul, 4125 Riehen (CH); Bopp, Urs, 4051 Basel (CH)
(72) Erfinder: SUHM, Norbert, D-79576 Wil-Haltingen (DE); MESSMER, Peter, CH-4104 Oberwil (CH); REGAZZONI, Pietro, CH-4054 Basel (CH); MÜLLER, Paul, CH-4125 Riehen (CH); BOPP, Urs, CH-4051 Basel (CH); HEHLI, Markus, CH-7276 Frauenkirch (CH); KOLLER, Silvio, CH-7270 Davos-Dorf (CH)
(74) Vertreter: Lusuardi, Werther Giovanni, Dr.
(86) Internationale Anmeldenummer: PCT/CH2000/000022
(87) Internationale Veröffentlichungsnummer: WO 2001/050959

(56) Entgegenhaltungen:
- EP-A- 0 253 333
- EP-A- 0 297 138
- EP-A- 0 910 990
- WO-A-00/32462
- WO-A-98/19875
- DE-A- 19 701 346
- US-A- 4 341 279
- US-A- 4 589 126
- US-A- 5 762 608
- PATENT ABSTRACTS OF JAPAN vol. 1999, no. 12, 29. Oktober 1999 (1999-10-29) & JP 11 188027 A (HITACHI MEDICAL CORP), 13. Juli 1999 (1999-07-13)
- PATENT ABSTRACTS OF JAPAN vol. 1997, no. 01, 31. Januar 1997 (1997-01-31) & JP 08 229028 A (HITACHI MEDICAL CORP), 10. September 1996 (1996-09-10)

## Beschreibung

Die Erfindung betrifft ein fahrbares Röntgengerät gemäss dem Oberbegriff des Patentanspruchs 1 wie es in der DE 197 01 346 A offenbart ist.

Röntgengeräte, insbesondere solche, die auf einem C-förmigen Bogen an einem Ende eine Röntgenstrahlenquelle und am anderen Ende einen Röntgenstrahlenempfänger (im folgenden kurz "C-Bogen" genannt) aufweisen, gehören zur Standardausstattung im unfallchirurgischen Operationssaal. Solche Geräte dienen der intraoperativen Bildgebung, um Repositionsmanöver bei den zu behandelnden Frakturen zu steuern und um das Einbringen von Implantaten für die Stabilisierung der Fraktur zu kontrollieren. Bei Anwendung des C-Bogens mit modernen chirurgischen Navigationssystemen werden intraoperativ erzeugte, in einem Computer speicherbare Fluoroskopiebilder benützt, um eine kontinuierliche Visualisierung des chirurgischen Verfahrens zu erlauben.

Da bei dem oben beschriebenen Vorgehen mit Röntgenstrahlen in Anwesenheit von Personen - Patient und OP Personal - gearbeitet wird, sollte die Positionierung des Röntgengerätes ohne lange Durchleuchtungszeit erfolgen und schon beim ersten Versuch richtig sein. Bislang wird aber der C-Bogen im Operationssaal nicht vom Operateur selbst, sondern durch einen OP Pfleger bewegt. Schon die Kommunikation zwischen dem Operateur und dem OP Pfleger über eine gewünschte, auszuführende Bewegung des Gerätes relativ zum Patienten beinhaltet zahlreiche Fehlermöglichkeiten. Oft wird der C-Bogen nach einem Operationsschritt benützt, um den Erfolg des Eingriffes zu kontrollieren und zu dokumentieren. Falls eine Korrektur des vorläufigen Ergebnisses erforderlich ist, muss der C-Bogen aus der mühsam gefundenen richtigen Position wieder entfernt werden, da das Gerät den Operateur sonst bei der Durchführung der Korrektur am Implantat oder am Knochenbruch behindert. Für die anschliessend notwendige Kontrolle muss der C-Bogen wiederum an die zuvor gefundene "richtige" Position dirigiert werden. Die Folge davon ist, dass oft lange Durchleuchtungszeiten entstehen, bis der Bildverstärker das richtige Bild liefert. Ein C-Bogen mit "Gedächtnis" für eine einmal gefundene korrekte Einstellung würde eine deutliche Verbesserung bedeuten.

Hier will die Erfindung Abhilfe schaffen. Der Erfindung liegt die Aufgabe zugrunde, eine Vorrichtung zur kontrollierten Bewegung eines medizinischen Geräts. insbesondere eines Röntgengerätes zu schaffen, dessen Antriebsvorrichungen "voice-controlled" steuerbar sind.

Die Erfindung löst die gestellte Aufgabe mit einem fahrbaren Röntgengerät, welches die Merkmale des Anspruchs 1 aufweist.

### Variante ohne chirurgisches Navigationssystem (Basisversion)

Damit ist der Vorteil erzielbar, dass die Bewegung des Röntgengerätes durch den Operateur selbst erfolgt. Die vom Operateur gegebenen und in seinem eigenen Koordinatensystem gedachten Anweisungen müssen dann nicht mehr von einer zweiten Person interpretiert und umgesetzt werden.

Das Röntgengerät umfasst mindestens drei, motorisch antreibbare Räder, welche über eine erste, zur Ebene parallel verlaufende, das Zentrum des Rades durchstossende Drehachse und eine zweite, senkrecht zur Ebene verlaufende, in der Ebene des Rades oder parallel zu dieser liegende Drehachse verfügen. Die Räder sind relativ zueinander fest positioniert. Jedes der Räder verfügt über eine erste Antriebsvorrichtung, mit welcher das Rad um die erste Drehachse rotierbar ist und über eine zweite Antriebsvorrichtung, mit welcher das Rad um die zweite Drehachse zur Änderung seiner Laufrichtung drehbar ist.

Die Räder können über ihre zweite Drehachse direkt am medizinischen Gerät befestigt sein, oder über ihre zweite Drehachse an einem als Plattform für das medizinische Gerät bestimmten Rahmen befestigt sein. Die Ausführung mit Plattform eignet sich besonders zur Nachrüstung bereits existierender Geräte, während die Ausführung mit direkt am Gerät befestigten Rädern eher für Neugeräte in Betracht zu ziehen ist.

Als erste und zweite Antriebsvorrichtungen sind Schrittmotoren, insbesondere elektronisch steuerbare Schrittmotoren einsetzbar. Ebenfalls sind Schrittmotoren auf der Basis der digitalen Antriebstechnik möglich. Weiterführende Angaben zu solchen Schrittmotoren können beispielsweise aus
Dubbel; Taschenbuch für den Maschinenbau; Hrsg. W. Beitz und K-H. Grote; Springer Verlag, 19. Auflage, 1997; Seiten: T9 und V29
entnommen werden.

Das erfindungsgemässe Röntgengerät ist vorzugsweise in Form eines C-Bogen mit einem Sockel auf der erfindungsgemässen Vorrichtung montiert. Bei C-Bogen kommen üblicherweise neben den oben erwähnten zwei Verfahrrichtungen in der Ebene parallel zum Boden des Operationsraumes weitere Bewegungsachsen für den C-Bogen relativ zum fahrbaren Sockel, welcher lediglich die Bewegungen des Gerätes parallel zum Boden ausführt, hinzu. Zur Handhabung des C-Bogens ist mindestens eine lineare Bewegung des C-Bogens parallel zu einer senkrecht auf dem Boden des Operationsraumes stehenden Achse (z-Achse) sowie Rotationbewegungen um mindestens zwei weitere Achsen notwendig. Auch diese Bewegungen des C-Bogens werden motorisiert ausgeführt. Der Antrieb dieser Bewegungen kann, wie vorangehend für die Räder des erfindungsgemässen Röntgengerätes beschrieben, ebenfalls mittels Schrittmotoren erfolgen. Für lineare Bewegungen sind Linearmotoren, wie sie beispielsweise in
Dubbel; Taschenbuch für den Maschinenbau; Hrsg. W. Beitz und K.-H. Grote; Springer Verlag, 19. Auflage, 1997; Seiten: T9 und V31
ausführlich beschrieben werden, geeignet.

Die Steuerung aller oben erwähnten Antriebe kann einerseits durch den Operateur unter direkter visueller Kontrolle oder durch Aufrufen von gespeicherten Daten bezüglich einer zuvor eingenommenen Position und Projektion erfolgen:
1. Die erste Positionierung des C-Bogens erfolgt in dieser Ausführungsform des erfindungsgemässen Röntgengerätes durch den Operateur unter direkter Sichtkontrolle mittels:
   a) Nutzen einer sterilisierbaren, vorzugsweise drahtlos arbeitenden Steuerkonsole; oder
   b) Voice controlled (z.B. mit den Befehlen: vor, zurück, rechts, links, schnell, langsam, schwenke um Achse A,B,C).
2. Zur Positionierung des C-Bogens durch Aufrufen von gespeicherten Daten umfasst das erfindungsgemässe Röntgengerät zusätzlich einen Computer mit mindestens einem Datenspeicher, einem Bildschirm und einer Tastatur. Zur Wiederholung einer zuvor eingenommenen Projektion ergibt sich somit die Möglichkeit in Kombination mit dem Computer die bei der ersten Positionierung durchfahrenen Wege in ihrer zeitlichen Abfolge und Länge im Datenspeicher zu speichern und durch Aufruf einer im Computer gespeicherten Steuerfunktion zu reproduzieren. Der Aufruf dieser Steuerfunktion kann wiederum mittels einer Steuerkonsole oder "voice-controlled" (z.B. mit den Befehlen: Position 1, Position 2) erfolgen.

Mit dem erfindungsgemässen Röntgengerät ist der Vorteil erzielbar, dass vom C-Bogen eingenommene Positionen und Projektionen speicherbar sind und durch Abrufen der gespeicherten Daten automatisch aus einer beliebigen Position wieder eingenommen werden können. Dies ist beispielsweise für Kontrollen nach Korrekturen an einem Implantat oder Knochenbruch, wozu der C-Bogen zwecks Nichtbehinderung des Operateurs aus seiner Position entfernt wird, günstig. Die Steuerung des C-Bogens erhält somit eine "Gedächtnisfunktion" bezüglich vorgängig eingestellter Projektionen. Intraoperativ bestimmte Positionen und Projektionen des C-Bogens bezüglich des Patienten lassen sich daher vom Operateur nach Durchführung weiterer Operationsschritte beliebig oft wieder reproduzieren, ohne dass erneute Durchleuchtungskontrollen für die Positionierung notwendig sind. Die computergesteuerte Ausführungsform ermöglicht zudem eine Visualisierung des geplanten Verfahrweges des C-Bogens auf dem Bildschirm des Computers, wodurch Kollisionen mit Gegenständen im Operationsraum beim automatischen Positionieren vermeidbar werden.

### C-Bogen mit kombiniertem chirurgischen Navigationssystem

In einer weiteren Ausführungsform des erfindungsgemässen Röntgengerätes wird dieses kombiniert mit einem chirurgischen Navigationssystem betrieben. Solche chirurgische Navigationssysteme umfassen mindestens einen Computer mit Datenspeicher und entsprechenden Bedienungselementen sowie eine vorzugsweise optoelektronische Positionserfassungsvorrichtung zur Ermittlung der Lage von optischen Markern innerhalb eines drei-dimensionalen Koordinatensystem im Operationsraum. Als chirurgisches Navigationssystem kann beispielsweise ein auf dem Markt erhältliches Gerät "Surgigate", welches von der Firma MEDIVISION, Oberdorf, Schweiz vertrieben wird, eingesetzt werden. Häufig werden solche Navigationssysteme mit einer optoelektronischen Positionserfassungsvorrichtung, beispielsweise einem im Handel erhältlichen Gerät Optotrak 3020 (three-dimensional motion measurement system) von Northern Digital, Ontario, Canada betrieben. Die Aufgabe des Steuerungscomputers für die Antriebsvorrichtungen des C-Bogens kann hier durch den im Navigationssystem integrierten Computer übernommen werden. Zur Erfassung der Position und Projektion des C-Bogens sind am C-Bogen mindestens drei nicht kollinear angeordnete, optische Marker, vorzugsweise LED (Light Emitting Dioden) oder Infrarot-LED angebracht. Die Lage der optischen Marker innerhalb des dreidimensionalen Koordinatensystems im Operationsraum wird durch die optoelektronische Positionserfassungsvorrichtung vermessen und die sich daraus ergebenden Koordinaten im Datenspeicher des Computers abgespeichert. Aus der Lage aller Marker kann dann durch den Computer die Position und Projektion des C-Bogens im Operationsraum berechnet werden.

Um den C-Bogen erstmals in eine gewünschte Position zu bringen, kann neben der Steuerung durch den Computer eine weitere Steuerung (ähnlich zur Ausführungsform ohne Kombination mit einem chirurgischen Navigationssystem) für die jeweils erste Primärpositionierung des C-Bogens in das System integriert sein. Als Optionen für diese weitere Steuerung ergeben sich:
a) Steuerung des C-Bogens durch Spracheingabe mittels der Stimme;
b) Steuerung des C-Bogens über eine sterile Steuerkonsole (Virtual Keyboard), welche im chirurgischen Navigationssystem integrierbar ist; und
c) Steuerung des C-Bogens mittels eines mit optischen Markern versehenen Pointers.

Der Pointer gemäss c) umfasst mindestens drei optische, nicht kollinear angeordnete Marker, vorzugsweise LED (Light Emitting Dioden) oder Infrarot-LED, welche an einem Stab angebracht sind. Der Pointer wird vom Operateur bezüglich des Patienten so ausgerichtet, dass die Längsachse des Stabes den zentralen Röntgenstrahl des C-Bogens simuliert. Zur Erfassung der Position und Orientierung des Pointers werden mittels der Vermessungsvorrichtung die optischen Marker bezüglich ihrer räumlichen Lage vermessen und im Computer anschliessend die Lage und Orientierung des Pointers bestimmt. Ebenfalls durch den Computer lässt sich eine Ebene im Raum bestimmen, welche senkrecht zur Längsachse des Pointers steht. Diese Ebene definiert die gewünschte Projektionsebene für die Primärpositionierung des C-Bogens. Anschliessend wird dem Röntgengerät der Befehl erteilt, die so definierte Position und Projektion einzunehmen.

Auch in der hier beschriebenen Ausführungsform können vom C-Bogen eingenommene Positionen und Projektionen im Computer abgespeichert werden; so dass durch späteres Wiederaufrufen der entsprechenden Daten vom C-Bogen zuvor eingenommene Positionen und Projektionen reproduzierbar sind.

Beim Einsatz eines chirurgischen Navigationssystemes mit Positionserfassungsvorrichtung besteht zusätzlich die Möglichkeit an einem Patienten eine Referenzbasis mit mindestens drei optischen, nicht kollinear angeordneten Markern zu befestigen. Dadurch können intraoperative Lageveränderungen des Patienten mittels Lagevermessung dieser Marker vermessen werden und die veränderte Position der Referenzbasis im Datenspeicher des Computers abgespeichert werden. Soll in der veränderten zweiten Position des Patienten im Operationsraum ein Röntgenbild aufgenommen werden, dessen Projektionsebene derjenigen eines in der ersten Position des Patienten aufgenommenen Röntgenbildes entspricht, kann mittels des Computers eine Koordinatentransformation durchgeführt werden und diejenige Position und Projektion des C-Bogens berechnet und vom C-Bogen angefahren werden, welche in der zweiten Position des Patienten die Aufnahme eines Röntgenbildes mit der zum gewählten, in der ersten Position aufgenommen Röntgenbild übereinstimmenden Projektionsebene ermöglicht. Auf diese Art lässt sich trotz möglicher Bewegungen der Referenzbasen, z.B. bei der Frakturreposition, die identische Bildebene wieder herstellen. Das heisst, diese Ausführungsform des erfindungsgemässen Röntgengerätes berücksichtigt bei der Wiederherstellung gespeicherter Projektionen mögliche Patientenbewegungen.

Für Kontrollen soll die zur einer vorherigen Position und Projektion des C-Bogens identische Position und Projektion wiederherstellbar sein. Dies ist durch Kombination des Computers mit der oben erwähnten weiteren Steuerung des C-Bogens möglich. Als Optionen für Eingabe der Steuerbefehle für diese weitere Steuerung ergeben sich auch hier:
a) Steuerung des C-Bogens durch Spracheingabe;
b) Steuerung des C-Bogens über eine sterile Steuerkonsole (Virtual Keyboard), welche im chirurgischen Navigationssystem integrierbar ist; und
c) Steuerung des C-Bogens mittels eines mit optischen Markern versehenen Pointers.

Die durch die Erfindung erreichten Vorteile sind im wesentlichen darin zu sehen, dass dank der erfindungsgemässen Vorrichtung der Bildverstärker im unfallchirurgischen Operationssaal wie bisher eingesetzt werden kann, dieser jedoch mit Hilfe der Steuerung solche Positionen einnimmt, die unmittelbar zur gewünschten Bildebene führen und diese z.B. nach Ausführen weiterer Operationsschritte aufgrund der Datenspeicherung beliebig oft für weitere Kontrollen wieder eingenommen werden kann.

Das Verfahren zur kontrollierten Bewegung eines medizinischen Röntgengerätes mit einer der oben beschriebenen Vorrichtungen umfasst die Verfahrensschritte:
A) Eingabe eines Befehls zur Bewegung des Gerätes in eine gewünschte Position durch den Bediener;
B) Verarbeiten dieses Befehls in Steuersignale durch eine Steuerungsvorrichtung;
C) Übertragen dieser Steuersignale an die an der Vorrichtung angebrachten Antriebsvorrichtungen;
D) Verfahren der bewegbaren Elemente in die gewünschte Position mittels der Antriebsvorrichtungen.

Die Eingabe des Befehls durch den Bediener kann mittels einer von Hand bedienbaren Steuerkonsole, als sprachliche Befehlseingabe oder über eine Tastatur oder eine Maus in einen Computer erfolgen.

Beim Einsatz eines Computers kann eine einmal eingenommene Position des Gerätes im Datenspeicher dieses Computers abgespeichert werden, wobei die Position des Gerätes durch Ermitteln der durchfahrenen Wege der bewegbaren Elemente in ihrer zeitlichen Abfolge und Länge im Computer berechenbar und im Datenspeicher abspeicherbar ist.

Anstelle der Berechnung der Position kann diese bei Anwendung einer optoelektronischen Positionserfassungsvorrichtung auch mittels Ausmessen der Lage von am Gerät angebrachten optischen Markern durch die optoelektronische Positionserfassungsvorrichtung im Datenspeicher abgespeichert werden.

Bei Kombination der Vorrichtung mit einem Computer ergibt sich somit der Vorteil, dass eine einmal eingenommene Position des Gerätes durch Aufruf einer Steuerfunktion und der im Datenspeicher abgespeicherten Daten wieder herstellbar ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung der fahrbaren Plattform für ein medizinisches Röntgengerät;
Fig. 2 eine vergrösserte perspektivische Darstellung eines Rades der Vorrichtung nach Fig. 1;
Fig. 3 eine Darstellung einer Gesamtvorrichtung, welche die Plattform nach Fig. 1 und das darauf gestellte medizinische Röntgengerät umfasst;
Fig. 4 eine perspektivische Darstellung einer computergesteuerten Gesamtvorrichtung; und
Fig. 5 eine perspektivische Darstellung einer Gesamtvorrichtung mit integrierter Positionserfassungsvorrichtung.

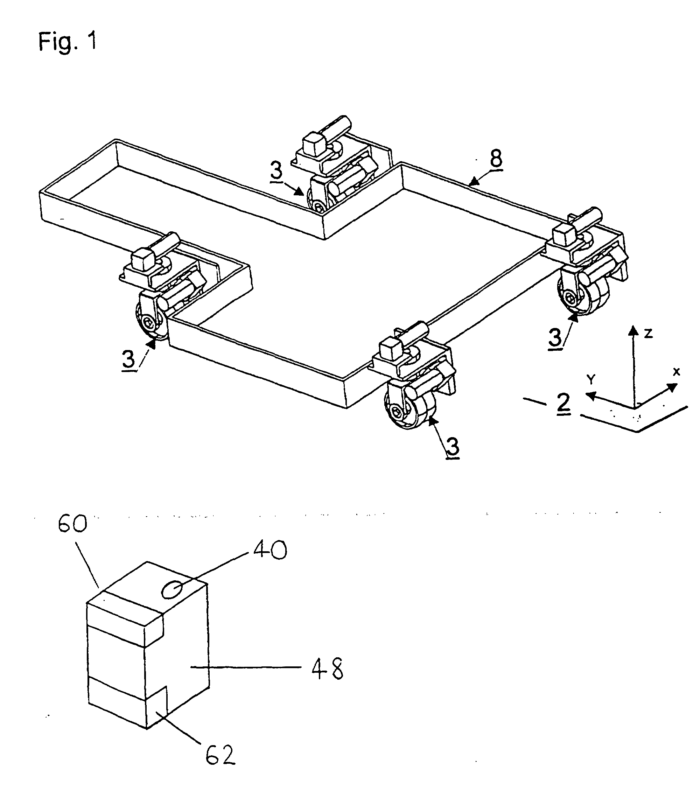

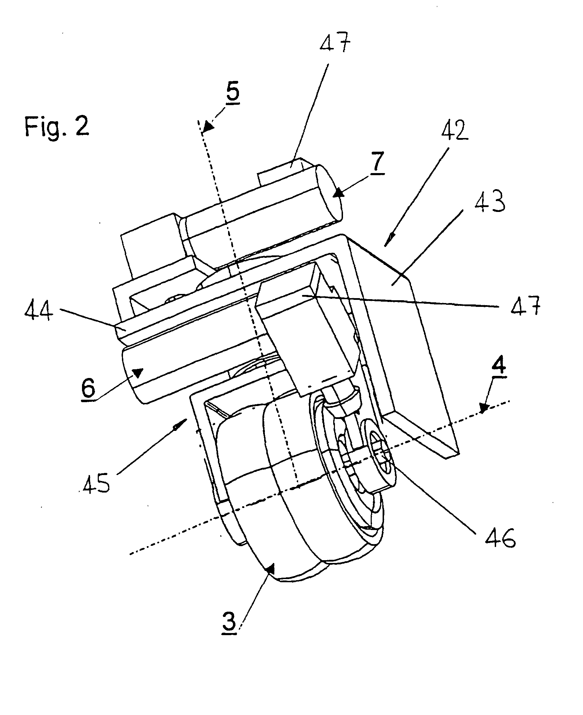

Fig. 1 und 2 zeigen die Vorrichtung zur kontrollierten Bewegung eines medizinischen Geräts in den zwei Dimensionen (x/y) einer Ebene 2, welche durch den Boden eines Operationssaals definiert ist. Ein ebener Rahmen 8 umfasst vier motorisch antreibbare Räder 3, welche über eine erste, zur Ebene 2 parallel verlaufende, das Zentrum des Rades 3 durchstossende Drehachse 4 und eine zweite, senkrecht zur Ebene 2 verlaufende, in der Ebene des Rades 3 liegende Drehachse 5 verfügen. Die Befestigung der Räder 3 am Rahmen 8 erfolgt über Winkelplatten 42, deren erster Plattenteil 43 am Rahmen 8 befestigt ist und in deren zweitem, rechtwinklig zum ersten Plattenteil 43 stehenden Plattenteil 44 die Radaufhängung 45 um die zweite Drehachse 5 rotierbar befestigt ist. An der Radaufhängung 45 sind die Radachsen 46 konzentrisch zur Drehachse 4 befestigt, so dass die mit den Radachsen 46 verbunden Räder 3 um die Drehachse 4 rotierbar sind. Die ersten Antriebsvorrichtungen 6, vorzugsweise Schrittmotoren, der Räder 3 sind radseitig und auf der Innenseite der Winkelplatte 42 mit den zweiten Plattenteilen 44 verbunden und ermöglichen eine Rotation der Räder 3 um deren erste Drehachse 4. Die zweiten Antriebsvorrichtungen 7, vorzugsweise Schrittmotoren, sind auf den von den Rädern 3 abgewandten Aussenseiten der Winkelplatten 42 mit den zweiten Plattenteilen 44 verbunden und ermöglichen eine Rotation der Radaufhängungen 45 und damit der Räder 3 um deren zweite Drehachse 5. An beiden Antriebsvorrichtungen 6;7 sind Empfänger 47 angebracht, welche die von einer Steuerung 48 ausgesendeten Steuersignale an die Antriebsvorrichtungen 6;7 übertragen. Die Steuerung 48 ist vorzugsweise fest programmierbar und über ein Mikrophon 40 voice-controlled bedienbar. Die vom Mikrophon 40 empfangenen Sprachsignale werden durch eine Spracherkennungseinheit 60 (voice-coder od. voice recognition unit) in Analogsignale oder digitale Signale gewandelt und an die Steuerung 48 übertragen. Die Steuerung 48 verarbeitet diese von der Spracherkennungseinheit 60 erhaltenen Signale in entsprechende Steuersignale für die Antriebsvorrichtungen 6;7 und übermittelt diese Steuersignale drahtlos mittels eines Senders 62 an die an den Antriebsvorrichtungen 6;7 angebrachten Empfänger 47. Anstelle eines einzelnen Senders 62, welcher die Steuersignale an alle Empfänger 47 überträgt, können auch mehrere Sender verwendet werden, wobei jeweils ein Sender zur Steuersignalübertragung an einen ihm zugeordneten Empfänger dient. Die Zuordnung kann beispielsweise durch Verwendung bestimmter Frequenzen erfolgen. Vorzugsweise bei Neugeräten besteht die Möglichkeit, anstelle einer drahtlosen Steuersignalübermittlung die Steuerung 48 komplett in die Vorrichtung oder das medizinische Gerät zu integrieren, so dass auch eine Übermittlung der Steuersignale von der Steuerung 48 an die Antriebsvorrichtungen 6;7 mittels Kabeln möglich ist.

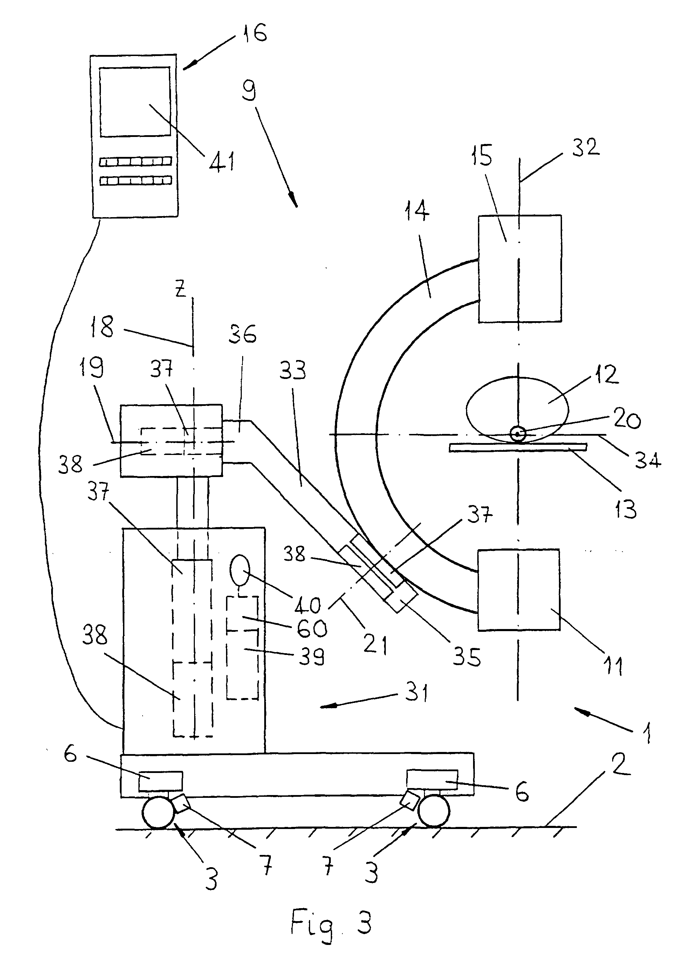

Fig. 3 zeigt eine Ausführungsform des erfindungsgemässen Röntgengerätes als C-Bogen 9 mit einem auf dem Boden des Operationsraumes fahrbaren Sockel 31, einer Röntgenstrahlenquelle 11 und einem Röntgenstrahlenempfänger 15, welche in einem Operationsraum um einen Operationstisch 13 angeordnet sind. Der fahrbare Sockel 31 ist in der Ebene 2, welche durch den Boden des Operationsraumes definiert ist, mittels der steuerbaren, am Sockel 31 über ihre zweite Drehachse 5 (Fig. 2) befestigten Räder 3 frei bewegbar, wodurch die Verschiebbarkeit des Röntgengerätes bezüglich zweier senkrecht zueinander stehender, in der Ebene 2 liegender Achsen (x;y) erzielt wird. Die Röntgenstrahlenquelle 11 und der Röntgenstrahlenempfänger 15 sind einander gegenüberliegend auf einer Zentralachse 32 angeordnet und mittels eines kreisbogenförmigen Trägers 14 fest verbunden und bilden den C-Bogen 9. Die Zentralachse 32 steht senkrecht zur Bildebene des aufzunehmenden Röntgenbildes und ebenso senkrecht zur Symmetrieachse 34 des Kreisbogens des Trägers 14. Der Träger 14 des C-Bogens 9 ist verschiebbar mit dem ersten Endstück 35 eines weiteren Verbindungsträgers 33 verbunden, wobei die Verschiebbarkeit zwischen Verbindungsträger 33 und dem C-förmigen Träger 14 entlang der äusseren Peripherie des C-förmigen Trägers 14 erfolgt und dadurch eine Rotation des C-Bogens 9 um eine Achse 20 ermöglicht wird, welche senkrecht auf einer durch die Symmetrieachse 34 und die Zentralachse 32 aufgespannten Ebene steht. Im Spezialfall der isozentrischen Bauweise liegt die Achse 20 im Zentrum des durch den Träger 14 definierten Halbkreises. Diese Bauweise weist zahlreiche Vorteile auf, u.a. dass ein einmal in den Bildmittelpunkt eingestelltes Objekt durch Rotationsbewegung um die Achse 20 nicht mehr aus dem Mittelpunkt herauswandert. Diese Bauform kommt jetzt mit neuen Geräten, beispielsweise bei dem Siremobil Iso-C von Siemens. Bei der nicht isozentrischen Bauweise liegt die Achse 20 auf der Symmetrieachse 34, jedoch ausserhalb des Mittelpunktes des Trägers 14, so dass die Strecke von der Achse 20 zum Träger 14 entlang der Symmetrieachse 34 länger ist als der Radius des Trägers 14. Dadurch ist der C-Bogen 9 austariert, d.h. er hat nicht die Tendenz, eine einmal eingestellte Projektion selbsttätig wieder zu verlassen durch Rotation des C-Bogens 9 um die Achse 20. Die Rotation des C-Bogens 9 relativ zum Verbindungsträger 33 ist ebenfalls nur bei einzelnen Bauformen vorhanden und erfolgt dann um eine bezüglich des Trägers 14 radiale, die Verbindung zwischen Verbindungsträger 33 und Träger 14 schneidende Achse 21. Der Verbindungsträger 33 ist mit seinem zweiten Endstück 36 mit dem fahrbaren Sockel 31 bewegbar verbunden. Die Bewegung des Verbindungsträgers 33 relativ zum fahrbaren Sockel 31 ist einerseits durch eine Verschiebbarkeit parallel zu einer senkrecht auf der Ebene 2 stehenden Achse 18 und eine Rotation um diese Achse 18 gegeben. Andererseits ist auch eine Verschiebbarkeit des Verbindungsträgers 33 parallel zu einer weiteren, parallel zur Ebene 2 verlaufenden Achse 19 sowie eine Rotation des Verbindungsträger 33 um diese Achse 19 möglich. Der Antrieb der bewegten Elemente erfolgt durch lineare Antriebsvorrichtungen 37 und rotative Antriebsvorrichtungen 6;7;38, welche über eine im Sockel 31 integrierte, vorzugsweise elektronische Steuerungsvorrichtung 39 steuerbar sind. Bedient wird die Steuerungsvorrichtung 39 voice-controlled über ein ebenfalls im Sockel 31 integriertes Mikrophon 40. Die Steuerungsvorrichtung 39 ist vorzugsweise fest programmierbar. Die vom Mikrophon 40 empfangenen Sprachsignale werden auch hier durch eine Spracherkennungseinheit 60 (voice-coder od. voice recognition unit) in Analogsignale oder digitale Signale gewandelt und an die Steuerungsvorrichtung 39 übertragen. Die Steuerungsvorrichtung 39 verarbeitet diese von der Spracherkennungseinheit 60 erhaltenen Signale in entsprechende Steuersignale für die Antriebsvorrichtungen 6;7;37;38 und übermittelt diese Steuersignale. Die Übertragung der Steuersignale von der Steuerungsvorrichtung 39 an die Antriebsvorrichtungen 6;7;37;38 erfolgt über Kabel. Das vom Röntgenstrahlenempfänger 15 empfangene Röntgenbild wird auf dem Bildschirm 41 eines Bildverstärkers 16 angezeigt. Als Antriebsvorrichtungen 6;7;37;38 werden vorzugsweise Schrittmotoren eingesetzt.

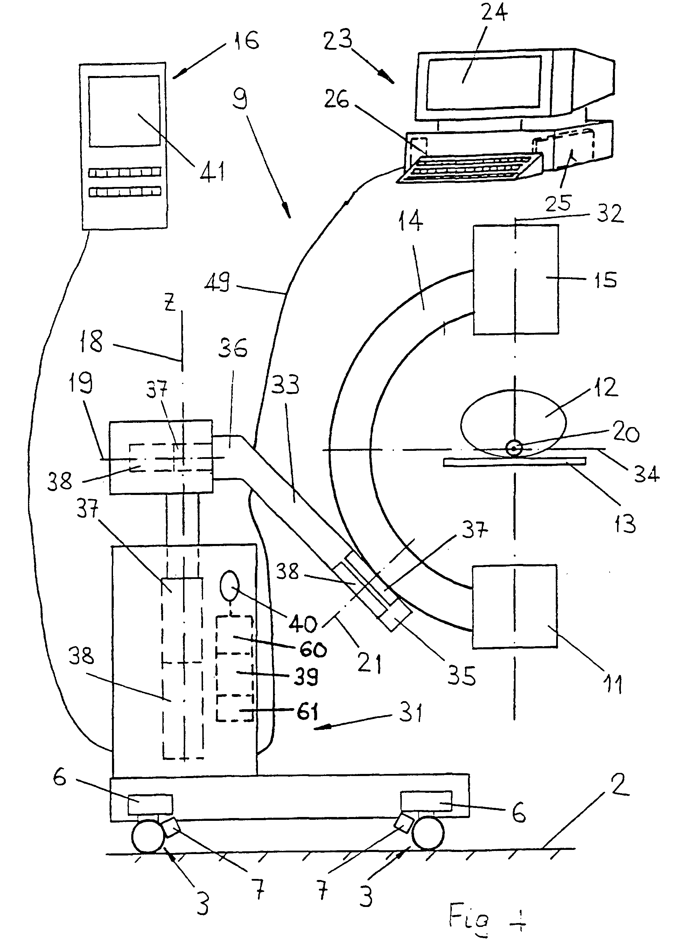

Die in Fig. 4 dargestellte Ausführungsform des erfindungsgemässen Röntgengerätes unterscheidet sich von der in Fig. 3 dargestellten Ausführungsform lediglich darin, dass die Steuerung der Antriebsvorrichtungen 6;7;37;38 am Sockel 31 und am Verbindungsträger 33 zusätzlich durch einen externen Computer 23 übernommen werden kann. Die Eingabe der Steuerbefehle kann über die Tastatur 26 oder eine Maus (nicht gezeichnet) erfolgen. Die Speicherung einmal eingenommener Positionen und Projektionen des C-Bogens 9 erfolgt im Datenspeicher 25 des Computers 23, so dass diese Positionen und Projektionen beliebig reproduzierbar sind. Die Übertragung der Steuersignale vom Computer 23 an die Antriebsvorrichtungen 6;7;37;38 erfolgt in dieser Ausführungsform über ein Kabel 49, welches den Computer 23 mit dem Sockel 31 verbindet. Um mögliche Kollisionen mit Gegenständen im Operationsraum zu vermeiden, kann der gewünschte Verfahrweg des Röntgengerätes zuvor am Bildschirm 24 des Computers 23 angezeigt werden. Verbunden mit dem Computer 23 ist eine analog zur in Fig. 3 beschriebenen Ausführungsform des erfindungsgemässen Röntgengerätes im Sockel 31 integrierte, vorzugsweise elektronische Steuerungsvorrichtung 39, womit die linearen Antriebsvorrichtungen 37 und rotativen Antriebsvorrichtungen 6;7;38 bezuglich ihrer Bewegung steuerbar sind. Bedient wird die Steuerungsvorrichtung 39 entweder durch Dateneingabe in den Computer 23 oder "voice-controlled" über ein ebenfalls im Sockel 31 integriertes Mikrophon 40. Die Steuerungsvorrichtung 39 ist vorzugsweise fest programmierbar. Die vom Mikrophon 40 empfangenen Sprachsignale werden auch hier durch eine Spracherkennungseinheit 60 (voice-coder od. voice recognition unit) in Analogsignale oder digitale Signale gewandelt und an die Steuerungsvorrichtung 39 übertragen. Die Steuerungsvorrichtung 39 verarbeitet diese von der Spracherkennungseinheit 60 erhaltenen Signale in entsprechende Steuersignale für die Antriebsvorrichtungen 6;7;37;38 und übermittelt diese Steuersignale. Die Übertragung der Steuersignale von der Steuerungsvorrichtung 39 an die Antriebsvorrichtungen 6;7;37;38 erfolgt über Kabel. Die erste Positionierung des C-Bogens 9 kann in dieser Ausführungsform durch Dateneingabe in den Computer 23 oder durch Sprachbefehle an die Steuerungsvorrichtung 39 erfolgen. Die eingenommene Position und Projektion des C-Bogens 9 wird durch den Computer 23 durch Berechnung der durchfahrenen Wege aller Antriebsvorrichtungen 6;7;37;38 in ihrer zeitlichen Abfolge und Länge ermittelt und im Datenspeicher 25 abgespeichert. Eine solche einmal eingenommene und abgespeicherte Position und Projektion des C-Bogens 9 kann später durch Aufruf einer Steuerfunktion reproduziert werden. Die Steuerfunktion kann in der Steuerungsvorrichtung 39 oder im Computer 23 programmiert sein und kann mittels Dateneingabe in den Computer 23 oder auch "voice-controlled" über die Steuerung 39 aufgerufen werden. Durch Aufruf dieser Steuerfunktion werden die im Datenspeicher 25 abgespeicherten, die Position und Projektion des C-Bogens 9 definierenden Daten aufgerufen und an die Steuerungsvorrichtung 39 übertragen, wodurch die gewünschte Position und Projektion des C-Bogens 9 wieder herstellbar ist. Als Variante der hier beschriebenen Ausführungsform des erfindungsgemässen Röntgengerätes ist auch eine Steuerung aller Antriebsvorrichtungen 6;7;37;38 nur durch den Computer 23 denkbar. Ebenfalls möglich ist eine vollständige Integration der Steuerungsvorrichtung 39 inklusive Spracherkennungseinheit 60 in den Computer 23.

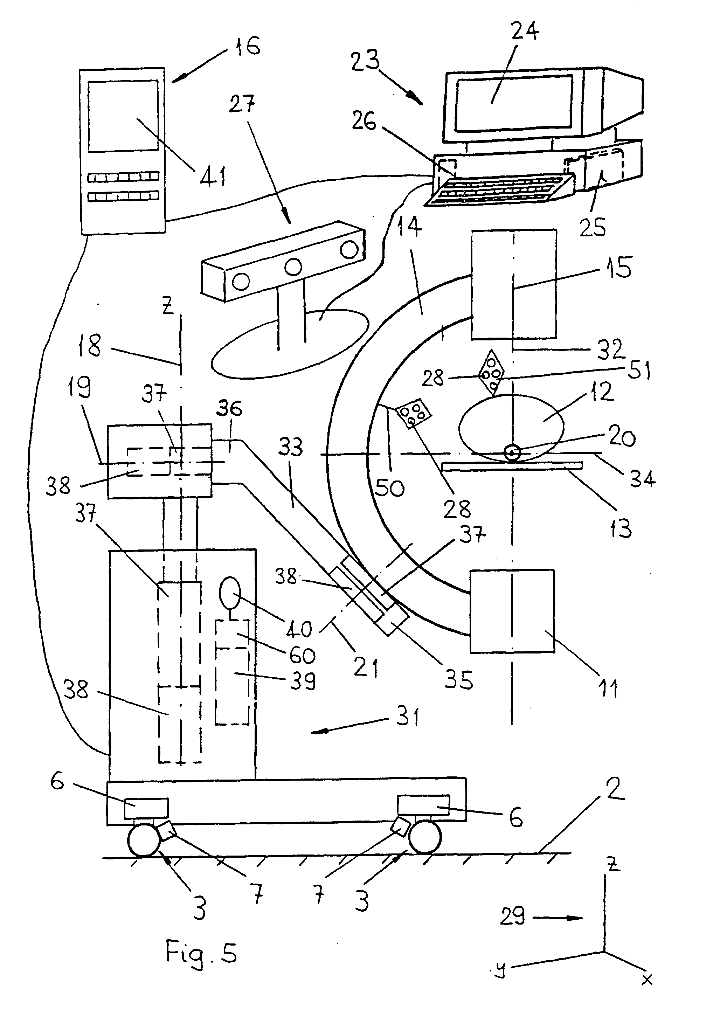

In Fig. 5 ist die mit einem chirurgischen Navigationssystem kombinierte Ausführungsform des erfindungsgemässen Röntgengerätes dargestellt. Die Unterschiede gegenüber der in Fig. 4 dargestellten Ausführungsform liegen lediglich darin, dass der C-Bogen 9 mit einer Referenzbasis 50 versehen ist. Eine weitere Referenzbasis 51 ist am Patienten 12 befestigt. Die Referenzbasen 50;51 umfassen je vier optische, nicht kollinear angeordnete Marker 28, welche durch LED ausgestaltet sind. Das chirurgische Navigationssystem umfasst eine optoelektronische Positionserfassungsvorrichtung 27, mittels welcher die Positionen der Marker 28 erfassbar und im Datenspeicher 25 die Lagekoordinaten der Marker 28 bezüglich eines dreidimensionalen Koordinatensystems 29 im Operationsraum speicherbar sind. Damit lassen sich Veränderungen der Position des Patienten 12 im Computer 23 erfassen. Durch den Computer 23 werden die gespeicherten Positionen und Projektionen des C-Bogens 9 so transformiert, dass in der veränderten zweiten Position des Patienten 12 diejenige Position und Projektion des C-Bogens berechnet und vom C-Bogen angefahren wird, welche die Aufnahme eines Röntgenbildes ermöglicht, dessen Projektionsebene mit derjenigen eines vorgängig in der ersten Position des Patienten 12 aufgenommenen Röntgenbildes übereinstimmt.

## Patentansprüche

1. Fahrbares Röntgengerät (1) mit
A) einem fahrbaren. Sockel (31) mit Rädern (3) und entsprechenden Antriebsvorrichtungen (6;7) zur translatorischen und rotativen Bewegung des Sockels (31) in den Achsrichtungen x und y eines Koordinatensystems (2;29);
B) einen relativ zum Sockel (31) in den Achsrichtungen x, y und z translatorisch und rotativ bewegbaren C-Bogen (9) mit einer Röntgenstrahlenquelle (22) und einem Röntgenstrahlenempfänger (15), welche mittels eines C-bogenförmigen Trägers (14) einander gegenüberliegend auf einer Zentralachse (32) angeordnet und fest verbunden sind, wobei
C) die Zentralachse (32) senkrecht zur Bildebene des aufzunehmenden Röntgenbildes steht; und
D) mindestens drei, motorisch antreibbare Räder (3) vorgesehen sind, welche über eine erste, zu einer Ebene (2) parallel verlaufende, das Zentrum des Rades (3) durchstossende Drehachse (4) und eine zweite, senkrecht zur Ebene (2) verlaufende, in der Ebene des Rades (3) oder parallel zu dieser liegende Drehachse (5) verfügen, und die Räder (3) zueinander in fester Relation positioniert sind;
E) jedes der Räder (3) über eine erste Antriebsvorrichtung (6) verfügt, mit welcher das Rad (3) um die erste Drehachse (4) rotierbar ist;
F) jedes der Räder (3) über eine zweite Antriebsvorrichtung (7) verfügt, mit welcher das Rad (3) um die zweite Drehachse (5) zur Änderung seiner Laufrichtung drehbar ist;
G) am Sockel (31) Antriebsvorrichtungen (37;38) zur Bewegung des C-Bogens (9) retativ zum Sockel (31) angebracht sind; und
H) das Röntgengerät (1) eine Steuerungsvorrichtung (39) umfasst, mittels welcher vom Bediener eingebbare Steuerbefehle bezüglich einer durch den C-Bogen (9) einzunehmenden Position und Projektion in entsprechende Steuersignale für die Antriebsvorrichtungen (6;7;37;38) umsetzbar sind,
**dadurch gekennzeichnet, dass**
I) das Röntgengerät (1) ein Mikrophon (40) und eine Spracherkennungseinheit (60) umfasst, wodurch die Steuerungsvorrichtung (39) "voice-controlled" bedienbar ist, wobei
K) die ersten und zweiten Antriebsvorrichtungen (6;7) der Räder (3) sowie die Antriebsvorrichtungen (37;38) zur Bewegung des C-Bogens (9) relativ zum Sockel (31) "voice-controlled" steuerbar sind.

2. Röntgengerät (1) nach Anspruch 1, **dadurch gekennzeichnet, dass** die Räder (3) über ihre zweite Drehachse (5) direkt am Röntgengerät (1) befestigt sind.

3. Röntgengerät (1) nach Anspruch 1, **dadurch gekennzeichnet, dass** die Räder (3) über ihre zweite Drehachse (5) an einem als Plattform für das Röntgengerät (1) bestimmten Rahmen (8) befestigt sind.

4. Röntgengerät (1) nach einem der Ansprüche 1 bis 3, **dadurch gekennzeichnet, dass** die ersten und zweiten Antriebsvorrichtungen (6;7) elektronisch steuerbare Schrittmotoren sind.

5. Röntgengerät (1) nach einem der Ansprüche 1 bis 4, **dadurch gekennzeichnet, dass** das Röntgengerät (1) einen Verbindungsträger (33) mit einem ersten Endstück (35) und einem longitudinal entgegengesetzten zweiten Endstück (36) umfasst, und das erste Endstück (35) bewegbar mit dem C-Bogen (9) und das zweite Endstück (36) bewegbar mit dem Sockel (31) verbunden ist.

6. Röntgengerät (1) nach Anspruch 5, **dadurch gekennzeichnet, dass** die Antriebsvorrichtungen (37;38) zur Bewegung des Verbindungsträgers (33) relativ zum Sockel (31) dienen und am Verbindungsträger (33) mindestens eine Antriebsvorrichtung (37) zur Bewegung des C-Bogens (9) relativ zum Verbindungsträger (33) angebracht ist.

7. Röntgengerät (1) nach Anspruch 5 oder 6, dass die äussere Peripherie des Trägers (14) durch ein Gleitlager tangential gleitbar mit dem ersten Endstück (35) verbunden ist.

8. Röntgengerät (1) nach einem der Ansprüche 5 bis 7, **dadurch gekennzeichnet, dass** das zweite Endstück (36) um eine senkrecht auf der Ebene (2) stehende Achse (18) verschiebar und rotierbar sowie um eine senkrecht zur Achse (18) stehende Achse (19) verschiebbar und rotierbar mit dem Sockel (31) verbunden ist.

9. Röntgengerät (1) nach einem der Ansprüche 1 bis 8, **dadurch gekennzeichnet, dass** sie zusätzlich eine von Hand bedienbare Steuerkonsole zur Bedienung der Steuerungsvorrichtung (39) umfasst.

10. Röntgengerät (1) nach einem der Ansprüche 1 bis 9, **dadurch gekennzeichnet, dass** es einen Computer (23) mit mindestens einem Datenspeicher (25), einem Bildschirm (24) und einer Tastatur (26) umfasst.

11. Röntgengerät (1) nach Anspruch 10, **dadurch gekennzeichnet, dass** die Antriebsvorrichtungen (6;7;37;38) mittels Dateneingabe in den Computer (23) automatisch durch diesen steuerbar sind und somit der C-Bogen (9) von einem Operateur durch Bedienung des Computers (23) kontrolliert verfahrbar ist.

12. Röntgengerät (1) nach Anspruch 10 oder 11, **dadurch gekennzeichnet, dass** die Position und Orientierung des C-Bogens (9) im Datenspeicher (25) des Computers (23) speicherbar sind.

13. Röntgengerät (1) nach einem der Ansprüche 10 bis 12, **dadurch gekennzeichnet, dass** im Datenspeicher (25) eine Steuerfunktion speicherbar ist, mittels welcher der C-Bogen (9) in eine im Datenspeicher (25) des Computers (23) gespeicherte Position und Orientierung des C-Bogens (9) reproduzierbar verfahrbar ist.

14. Röntgengerät (1) nach Anspruch 13, **dadurch gekennzeichnet, dass** der Computer (23) mit der Steuerungsvorrichtung (39) verbunden ist und der Aufruf dieser Steuerfunktion mittels einer vom Operateur bedienbaren Steuerkonsole erfolgt.

15. Röntgengerät (1) nach Anspruch 14, **dadurch gekennzeichnet, dass** der Computer (23) mit der Steuerungsvorrichtung (39) verbunden ist und der Aufruf dieser Steuerfunktion "voice-controlled" erfolgt.

16. Röntgengerät (1) nach einem der Ansprüche 1 bis 15, **dadurch gekennzeichnet, dass** es mit einem chirurgischen Navigationssystem kombiniert ist, welches eine optoelektronische Positionserfassungsvorrichtung (27) umfasst.

17. Röntgengerät (1) nach Anspruch 16, **dadurch gekennzeichnet, dass** zusätzlich mindestens drei, nicht kollinear angeordnete optische Marker (28) am C-Bogen (9) befestigt sind, so dass die Position der Marker (28) durch die optoelektronische Positionserfassungsvorrichtung (27) erfassbar und im Datenspeicher (25) die Lagekoordinaten der Marker (28) bezüglich eines dreidimensionalen Koordinatensystemes (29) im Operationsraum speicherbar sind.

18. Röntgengerät (1) nach Anspruch 16 oder 17, **dadurch gekennzeichnet, dass** es zusätzlich mindestens eine, an einem Patienten (12) befestigbare Referenzbasis (51) umfasst, welche mindestens drei optische, nicht kollinear angeordnete Marker (28) enthält, so dass die Position der Marker (28) durch die optoelektronische Positionserfassungsvorrichtung (27) erfassbar und im Datenspeicher (25) die Lagekoordinaten der Marker (28) bezüglich eines dreidimensionalen Koordinatensystems (29) im Operationsraum speicherbar ist.

19. Röntgengerät (1) nach einem der Ansprüche 1 bis 18, **dadurch gekennzeichnet, dass** die Antriebsvorrichtungen (37) Linearschrittmotoren sind.

20. Röntgengerät (1) nach einem der Ansprüche 1 bis 19, **dadurch gekennzeichnet, dass** die Antriebsvorrichtungen (38) Schrittmotoren sind.

21. Röntgengerät (1) nach Anspruch 19 oder 20, **dadurch gekennzeichnet, dass** die Schrittmotoren digital ansteuerbar sind.

## Claims

1. A movable X-ray apparatus (1) including
A) a movable pedestal (31) provided with rollers (3) and corresponding drive devices (6;7) permitting a translatory and a rotational movement of the pedestal (31) in the axial directions x and y of a coordinate system (2;29);
B) a C-bow (9) capable of translatory and rotational movement relative to the pedestal (31) in the axial directions x, y, and z, including an X-ray source (22) and an X-ray receiver (15) which are arranged and fixedly connected in opposite positions on a central axis (32) by means of a support (14) shaped in the form of C-bow, whereby
C) the central axis (32) extends vertically to the image plane of the X-ray photograph to be taken; and
D) at least three motor-driven rollers (3) which are provided with a first axis of rotation (4) extending parallel to a plane (2) and intersecting the centre of the roller (3), and with a second axis of rotation (5) extending perpendicularly to said plane (2) and lying in the plane of the roller (3) or parallel thereto, the rollers (3) being positioned in a fixed relationship relative to one another,
E) each of the rollers (3) is provided with a first drive device (6) by means of which the roller (3) is rotatable about the first axis of rotation (4); and
F) each of the rollers (3) is provided with a second drive device (7) by means of which the roller (3) is rotatable about the second axis of rotation (5) so as to change its direction of travel;
G) drive devices (37;38) mounted on the pedestal (31) for moving the C-bow (9) relative to the pedestal (31); and
H) the X-ray apparatus (1) including a control device (39) by means of which control instructions entered by the operator concerning a particular position and projection to be occupied by the C-bow (9) may be converted into corresponding control signals to be transmitted to the drive devices (6;7;37;38),
**characterised in that**
I) the X-ray apparatus (1) comprises a microphone (40) and a voice recognition unit (60) making it possible to operate the control unit (39) via voice control, whereby
K) that the first and second drive devices (6;7) of the rollers (3) as well as the drive devices for moving the C-bow (9) relative to the pedestal (31) are controllable via voice control.

2. An X-ray apparatus (1) as claimed in claim 1, **characterised in that** the rollers (3) are fixed directly to the medical apparatus (1) via their second axis of rotation (5).

3. An X-ray apparatus (1) as claimed in claim 1, **characterised in that** via their second axis of rotation (5) the rollers (3) are fixed to a frame (8) designed to serve as a platform for the medical apparatus (1).

4. An X-ray apparatus (1) as claimed in any of the claims 1 to 3, **characterised in that** the first and the second drive devices (6;7) are realised in the form of electronically controllable stepper motors.

5. An X-ray apparatus (1) as claimed in any of the claims 1 to 4, **characterised in that** it comprises a connecting support (33) having a first end piece (35) and, longitudinally opposite thereto, a second end piece (36) and that the first end piece (35) is movably connected to the C-bow (9) while the second end piece (36) is movably connected to the pedestal (31).

6. An X-ray apparatus as claimed in claim 5, **characterised in that** the drive devices (37;38) permit a displacement of the connecting support (33) relative to the pedestal (31) and that the connecting support (33) is provided with at least one drive device (37) permitting a displacement of the C-bow (9) relative to the connecting support (33).

7. An X-ray apparatus (1) as claimed in claim 5 or 6, **characterised in that** the outer periphery of the support (14) is connected to the first end piece (35) by means of a slide bearing so as to be tangentially slidable.

8. An X-ray apparatus (1) as claimed in any of the claims 5 to 7, **characterised in that** the second end piece (36) is connected to the pedestal (31) in such a way as to be displaceable along and rotatable about an axis (18) extending vertically to the plane (2) and about an axis (19) extending vertically to the axis (18).

9. An X-ray apparatus (1) as claimed in any of the claims 1 to 8, **characterised in that** it comprises in addition a hand-operated control console designed for manipulating the control device (39).

10. An X-ray apparatus (1) as claimed in any of the claims 1 to 9, **characterised in that** it comprises in addition a computer (23) including at least a data memory (25), a display screen (24), and a keyboard (26).

11. An X-ray apparatus (1) as claimed in claim 10, **characterised in that** the drive devices (6;7;37;38) are automatically controllable by the computer (23) by means of data input into said computer and that the C-bow may thus be displaced in a controlled manner by an operating surgeon by entering data into the computer (23).

12. An X-ray apparatus (1) as claimed in claim 10 or 11, **characterised in that** the position and orientation of the C-bow (9) may be stored in the data memory (25) of the computer (23).

13. An X-ray apparatus (1) as claimed in any of the claims 10 to 12, **characterised in that** a control function may be stored in the data memory (25) by means of which the C-bow (9) may be displaced in such a way as to reproduce any given position and orientation of the C-bow (9) stored in the data memory (25) of the computer (23).

14. An X-ray apparatus (1) as claimed in claim 13, **characterised in that** the computer (23) is connected to the control device (39) and that the activation of the control function is realised by means of a control console to be manipulated by the operating surgeon.

15. An X-ray apparatus as claimed in claim 14, **characterised in that** the computer (23) is connected to the control device (39) and that the activation of the control function is realised by means of voice control.

16. An X-ray apparatus (1) as claimed in any of the claims 1 to 15, **characterised in that** it is combined with a surgical navigation system which comprises an optoelectronic position detector (27).

17. An X-ray apparatus (1) as claimed in claim 16, **characterised in that** in addition at least three optical markers (28) are fixed to the C-bow (9) which are arranged in a non-collinear manner, so that the positions of the markers (28) may be determined by the optoelectronic position detector (27) and the position coordinates of the markers (28) relative to a three-dimensional coordinate system (29) extending throughout the operating theatre may be stored in the data memory (25).

18. An X-ray apparatus (1) as claimed in claim 16 or 17, **characterised in that** it comprises in addition at least one reference base (51) to be applied to a patient (12) which includes at least three optical markers (28) arranged in a non-collinear manner, so that the position of the markers (28) may be determined by the optoelectronic position detector (27) and the position coordinates of the markers (28) relative to a three-dimensional coordinate system (29) extending throughout the operating theatre may be stored in the data memory (25).

19. An X-ray apparatus (1) as claimed in any of the claims 1 to 18, **characterised in that** the drive devices (37) are realised as linear stepper motors.

20. An X-ray apparatus (1) as claimed in any of the claims 1 to 19, **characterised in that** the drive devices (38) are realised as stepper motors.

21. An X-ray apparatus (1) as claimed in claim 19 or 20, **characterised in that** the stepper motors may be digitally controlled.

## Revendications

1. Appareil de radiologie mobile (1) comportant
A) un socle mobile (31) avec des roues (3) et des dispositifs d'entraînement correspondants (6;7) permettant un mouvement de translation et de rotation du socle (31) dans les directions axiales x et y d'un système de coordonnées (2;29);
B) un arc en C (9) pouvant effectuer un mouvement de translation et de rotation dans les directions axiales x, y et z par rapport au socle (31) et comportant une source de rayons X (22) et un récepteur de rayons X (15), lesquels sont, au moyen d'un support (14) en forme d'arc en C, disposés l'un en regard de l'autre sur un axe central (32) et reliés entre eux de manière fixe,
C) l'axe central (32) étant situé perpendiculairement au plan de la radiographie à prendre, et
D) au moins trois roues (3) pouvant être actionnées par moteur étant prévues, lesquelles disposent d'un premier axe de rotation (4) s'étendant parallèlement à un plan (2) et traversant le centre de la roue (3), ainsi qu'un deuxième axe de rotation (5) s'étendant perpendiculairement au plan (2) et situé dans le plan de la roue (3) ou parallèlement à celui-ci, et les roues (3) étant positionnées dans une relation fixe les unes par rapport aux autres;
E) chacune des roues (3) disposant d'un premier dispositif d'entraînement (6) permettant de faire tourner la roue (3) sur le premier axe de rotation (4);
E) chacune des roues (3) disposant d'un deuxième dispositif d'entraînement (7) permettant de faire tourner la roue (3) sur le deuxième axe de rotation (5) afin de changer son sens de déplacement;
G) sur le socle (31) étant fixés des dispositifs d'entraînement (37;38) destinés au déplacement de l'arc en C (9) par rapport au socle (31); et
H) l'appareil de radiologie (1) comprenant un dispositif de commande (39) au moyen duquel des instructions de commande entrées par l'opérateur et relatives à une position et une projection devant être prise par l'arc en C (9) peuvent être converties en signaux de commande destinés aux dispositifs d'entraînement (6;7;37;38),
**caractérisé en ce que**
i) l'appareil de radiologie (1) comprend un microphone (40) et une unité de reconnaissance vocale (60), ce qui fait que le dispositif de commande (39) peut être commandé par contrôle vocal ("voice-controlled"),
K) les premier et deuxième dispositifs d'entraînement (6;7) des roues (3) ainsi que les dispositifs d'entraînement (37;38) destinés au déplacement de l'arc en C (9) par rapport au socle (31) pouvant être commandés par contrôle vocal.

2. Appareil de radiologie (1) selon la revendication 1, **caractérisé en ce que** les roues (3) sont fixées directement sur l'appareil de radiologie (1) par l'intermédiaire de leur deuxième axe de rotation (5).

3. Appareil de radiologie (1) selon la revendication 1, **caractérisé en ce que** les roues (3) sont fixées, par le biais de leur deuxième axe de rotation (5), à un cadre (8) servant de plate-forme à l'appareil de radiologie (1).

4. Appareil de radiologie (1) selon l'une des revendications 1 à 3, **caractérisé en ce que** les premiers et deuxièmes dispositifs d'entraînement (6;7) sont des moteurs pas à pas commandables de manière électronique.

5. Appareil de radiologie (1) selon l'une des revendications 1 à 4, **caractérisé en ce que** l'appareil de radiologie (1) comprend un support de liaison (33) avec une première extrémité (35) et, longitudinalement opposée à celle-ci, une deuxième extrémité (36), et que la première extrémité (35) est reliée de manière mobile à l'arc en C et la deuxième extrémité est reliée de manière mobile au socle (31).

6. Appareil de radiologie (1) selon la revendication 5, **caractérisé en ce que** les dispositifs d'entraînement (37;38) servent au déplacement du support de liaison (33) par rapport au socle (31) et que sur le support de liaison (33) est monté au moins un dispositif d'entraînement (37) destiné au déplacement de l'arc en C (9) par rapport au support de liaison (33).

7. Appareil de radiologie (1) selon la revendication 5 ou 6, **caractérisé en ce que** la périphérie extérieure du support (14) est reliée par un palier à glissement à la première extrémité (35) de manière à pouvoir glisser tangentiellement par rapport à celle-ci.

8. Appareil de radiologie (1) selon l'une des revendications 5 à 7, **caractérisé en ce que** la deuxième extrémité (36) est reliée au socle (31) de manière à pouvoir se déplacer et tourner sur un axe (18) situé perpendiculairement au plan (2) et de manière à pouvoir se déplacer et tourner sur un axe (19) situé perpendiculairement à l'axe (18).

9. Appareil de radiologie (1) selon l'une des revendications 1 à 8, **caractérisé en ce qu'**il comprend en outre une console de commande pouvant être manipulée manuellement et servant à commander le dispositif de commande (39).

10. Appareil de radiologie (1) selon l'une des revendications 1 à 9, **caractérisé en ce qu'**il comprend un ordinateur (23) avec au moins une mémoire de données (25), un écran (24) et un clavier (26).

11. Appareil de radiologie (1) selon la revendication 10, **caractérisé en ce que** les dispositifs d'entraînement (6;7;37;38) sont, au moyen de l'entrée de données dans l'ordinateur (23), commandables de manière automatique par l'intermédiaire de celui-ci et que l'arc en C peut donc être déplacé de manière contrôlée par un chirurgien au moyen de l'ordinateur (23).

12. Appareil de radiologie (1) selon la revendication 10 ou 11, **caractérisé en ce que** la position et l'orientation de l'arc en C (9) sont stockables dans la mémoire de données (25) de l'ordinateur (23).

13. Appareil de radiologie (1) selon l'une des revendications 10 à 12, **caractérisé en ce que** dans la mémoire de données (25) peut être stockée une fonction de commande, grâce à laquelle l'arc en C (9) peut être déplacé de manière reproductible dans une position et une orientation stockées dans la mémoire de données (25) de l'ordinateur (23).

14. Appareil de radiologie (1) selon la revendication 13, **caractérisé en ce que** l'ordinateur (23) est relié au dispositif de commande (39) et **en ce que** l'appel de cette fonction de commande s'effectue au moyen d'une console de commande pouvant être manipulée par le chirurgien.

15. Appareil de radiologie (1) selon la revendication 14, **caractérisé en ce que** l'ordinateur (23) est relié au dispositif de commande (39) et **en ce que** l'appel de cette fonction de commande s'effectue par contrôle vocal ("voice-controlled").

16. Appareil de radiologie (1) selon l'une des revendications 1 à 15, **caractérisé en ce qu'**il est combiné avec un système de navigation chirurgical qui comprend un dispositif de saisie de position optoélectronique (27).

17. Appareil de radiologie (1) selon la revendication 16, **caractérisé en ce qu'**au moins trois marqueurs optiques (28) disposés de manière non-colinéaire sont en outre fixés sur l'arc en C (9), de sorte que la position des marqueurs (28) peut être saisie par le dispositif de saisie de position optoélectronique (27) et **en ce que** les coordonnées de position des marqueurs (28) peuvent être stockées dans la mémoire de données (25) par rapport à un système de coordonnées tridimensionnel représentant la salle d'opération.

18. Appareil de radiologie (1) selon la revendication 16 ou 17, **caractérisé en ce qu'**il comprend en outre au moins une base de référence (51) pouvant être fixée sur un patient (12), laquelle comporte au moins trois marqueurs optiques (28) disposés de manière non-colinéaire, de sorte que la position des marqueurs (28) peut être saisie par le dispositif de saisie de position optoélectronique (27) et que les coordonnées de position des marqueurs (28) peuvent être stockées dans la mémoire de données (25) par rapport à un système de coordonnées tridimensionnel (29) représentant la salle d'opération.

19. Appareil de radiologie (1) selon l'une des revendications 1 à 18, **caractérisé en ce que** les dispositifs d'entraînement (37) sont des moteurs pas à pas linéaires.

20. Appareil de radiologie (1) selon l'une des revendications 1 à 19, **caractérisé en ce que** les dispositifs d'entraînement (38) sont des moteurs pas à pas.

21. Appareil de radiologie (1) selon la revendication 19 ou 20, **caractérisé en ce que** les moteurs pas à pas peuvent être activés de manière numérique.
